# EUROPEAN PATENT APPLICATION

(11) **EP 4 296 676 A1**
(43) Date of publication of application: **27.12.2023**
(21) Application number: 22180465.1
(22) Date of filing: 22.06.2022
(51) Int. Cl.: G01N 33/68, G16B 40/00, G16H 50/30

(54) **METHOD FOR PREDICTION OF SURVIVAL RATE AND ITS USE**

(71) Applicant: Mosaiques Diagnostics And Therapeutics AG, 30659 Hannover (DE)
(72) Inventor: Mischak, Harald, Hannover (DE)
(74) Representative: Kröncke, Rolf

(57) **Abstract**

The present invention relates in a first aspect to a method for the prediction of a survival rate of an individual within a predetermined time period or at a predetermined time end point, whereby a body fluid sample from the individual is analysed and based on the information of peptidome/proteome present in said body fluid, the survival rate of said individual within the predetermined time period or at a predetermined time end point is calculated. Further, the present invention applies a specific predetermined multidimensional classifier for calculating the survival rate. That is, the survival rate reflects the probability of future death within the predetermined time period or the predetermined time end point. The method includes in an embodiment artificial intelligence routine. The individuals analysed include individuals with pre-existing conditions as well as healthy individuals. Moreover, a computer implemented method for the prediction of the survival rate of an individual is provided as well as a computer readable medium or computer program product having computer executable instructions for performing the method according to the present invention.

## Description

The present invention relates in a first aspect to a method for the prediction of a survival rate of an individual within a predetermined time period or at a predetermined time end point, whereby a body fluid sample from the individual is analysed and based on the information of peptidome/proteome present in said body fluid, the survival rate of said individual within the predetermined time period or at a predetermined time end point is calculated. Further, the present invention applies a specific predetermined multidimensional classifier for calculating the survival rate. That is, the survival rate reflects the probability of future death within the predetermined time period or the predetermined time end point. The method includes in an embodiment artificial intelligence routine. The individuals analysed include individuals with pre-existing conditions as well as healthy individuals. Moreover, a computer implemented method for the prediction of the survival rate of an individual is provided as well as a computer readable medium or computer program product having computer executable instructions for performing the method according to the present invention.

### Prior art

Urine contains more than 20,000 endogenous peptides or proteins which are partly generated along the nephron all which pass up into the tubular fluid through the glomerular barrier. The value of the urinary peptidomic profile has been demonstrated in various publications and for various approaches. For example, the urinary peptidomic profile predicts outcome in SARS-CoV-2 infected patients, see Wendt, R., et al., E Clinical Medicine 2021;36:100883. That is sequencing these urinary peptides identifies parental proteins and the urinary peptidomic profile (UPP) provides a body wide molecular signature of ongoing pathophysiological processes. For example, UPP generated molecular signatures have been shown to be associated with a preclinical phase of heart failure, chronic kidney disease, diabetic nephropathy and a variety of other disorders, see e. g. Chang, Z. J. Y., et al., Proteomics Clin Appl, 2019, 13, E1800174 or Chang Z. J. Y., et al., 2015, Hypertension, 66, 52 to 60. For example, Martens D. S., et al., doi.org/10.106/s2666-7568 (21)00226-9 refers to urinary peptidomic profiles to address age related disabilities and provides a prospective population study to identify and validate a UPP signature that differentiates healthy from unhealthy agents in a population cohort with long term follow up; to replicate the trained UPP agent clocking patients and validate the clock in the general population as a correlate or predictor of adverse health outcomes; and to detect the molecular pathways implicated in unhealthy agents.

In particular, Martens et al. identify that the UPP signature described therein is indicative of aging, reflects fibrosis and extracellular matrix modelling, and was associated with risk factors and adverse health outcomes in the population and with accelerated aging in patients. Further, He. T. et al., Clin. Transl. Med., 2021, 11, E267, identifies serum and urinary biomarkers of collagen type I turnover predicting prognosis in patients with heart failure.

However, there is still an ongoing need for methods enabling predictions of the survival rate of individuals within a predetermined time period or at a predetermined time end point, in particular, in case of healthy individuals prospective predictability of adverse events, in particular, death, is desired to allow preventive measures including pharmaceutical and physical interference.

### Brief description of the present invention

In a first aspect the present invention relates to a method for the prediction of the survival rate of an individual within a predetermined time period or at a predetermined time end point, comprising the steps of
a) Providing a body fluid sample from the individual;
b) Determining the body fluid peptidome/proteome in the urinary sample;
c) Comparing the information on the body fluid peptidome/proteome obtained in step b) with information on the body fluid peptidome/proteome present in a database;
d) Determining the survival rate of said individual within the predetermined time period or at the predetermined end point based on the comparison in step c).

In particular, the method allows to predict the survival rate or in other terms the adverse effect of death at a predetermined time period or a predetermined time end point in an individual suffering from a pre-existing disease.

In a further aspect, the method is based on determining the scoring of a specific predefined multidimensional classifier, e. g. by artificial intelligence routine, to predict future death in an individual within the predetermined time period or at the predetermined time end point. Further, the present invention provides a computer implemented method for the prediction of the survival rate of an individual, comprising the steps of
a) Obtaining information of the peptidome/protein in a sample obtained from said individual;
b) Computing the information of step a) by comparing the information of the peptidome/proteome obtained in step a) with information on the peptidome/proteome obtained from a database; and
c) Determining the survival rate of said individual within a predetermined time period or a predetermined time end point respectively;
d) Optionally, further comprising the step of showing the data of prediction of the survival rate of an individual on an output unit.

Finally, a computer readable medium or computer program product having computer executable instructions for performing the steps according to the present invention is provided.

### Brief description of the figures

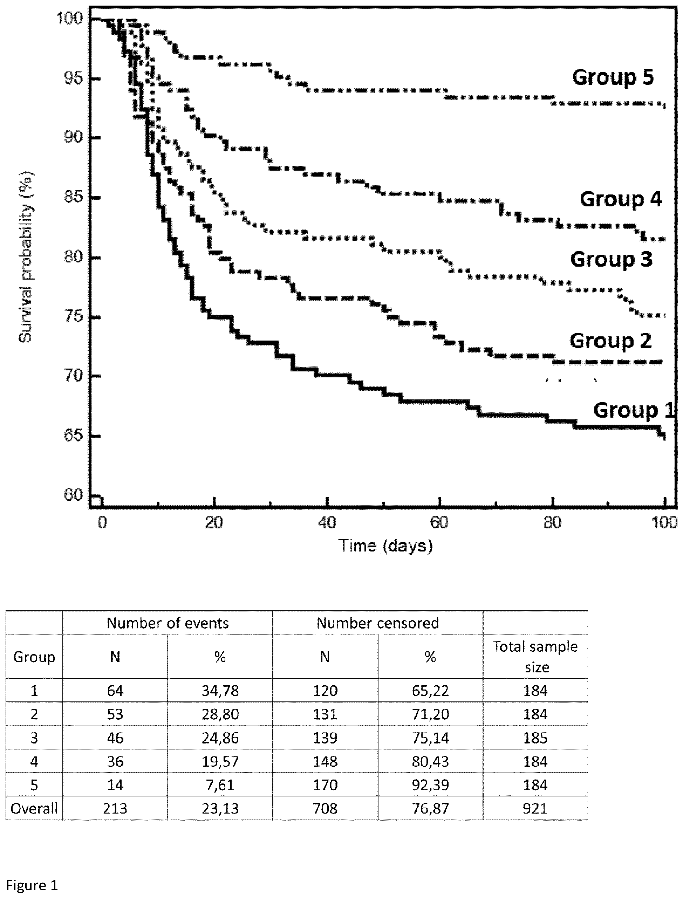

Figure 1 depicts the association of the scoring of a predictive multidimensional classifier with the survival probability over time of a cohort of 8271 participants according to the present invention, whereby the participants include apparently healthy subject and patients with different chronic disease, including diabetes, hypertension, chronic kidney disease, cardiovascular disease, and different types of cancer. The table of figure 1 shows the survival probability of the different quintiles, separated as a result of the classification based on the classifier. A highly significant continuous decrease of survival probability with decreasing score is evident.

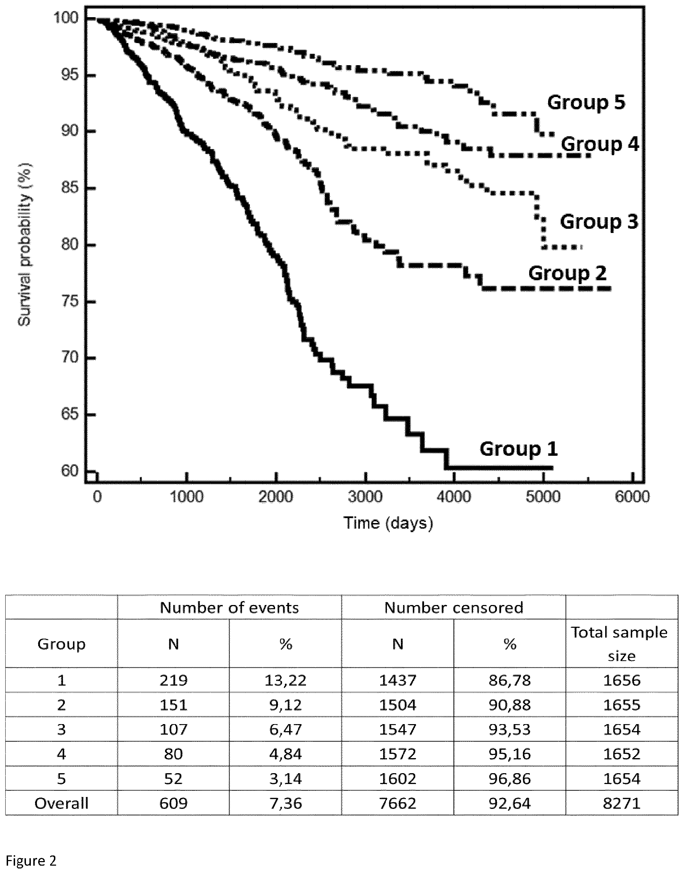

Figure 2 depicts the survival analysis of 921 ICU patients as described in example 2 with similar separation into five groups according to the classifier scoring, showing the survival probability for each of the classified groups accordingly.

### Detailed description of the present invention

In a first aspect, the present invention relates to a method for the prediction of the survival rate of an individual within a predetermined time period or at a predetermined time end point, comprising the steps of
a) Providing a body fluid sample from the individual;
b) Determining the body fluid peptidome/proteome in the urinary sample;
c) Comparing the information on the body fluid peptidome/proteome obtained in step b) with information on the body fluid peptidome/proteome present in a database;
d) Determining the survival rate of said individual within the predetermined time period or at the predetermined end point based on the comparison in step c).

In this connection, the term "predetermined time period" refers to a predefined period of time being predefined in e. g. a period of days, months or years. For example, the predetermined time period may be a period within the next five years, or three years or one year, etc.

In addition, the term "predetermined time end point" refers to a specific time end point, e. g. at five years after the evaluation by the method according to the present invention. Also the predetermined time end point can be expressed as days, months or years depending on the approach applied.

Further, the term "peptidome/proteome" refers to all detectable proteins and peptides in a sample with a molecular mass between 800 and 20000 Da.

In addition, the term "survival rate" expresses the probability, typically in percent or as a score, being alive at the predetermined time end point or staying alive within the predetermined time period. In other words, the survival rate also identifies a possible future death in percentage accordingly. As said, the survival rate is typically identified in a percentage of survival rate or in reversed expression as percentage of decease.

As used herein, the term "classifier" refers to a numeric variable that is based on a multitude of predefined peptides or proteins determinable in a urinary sample. The classifier has been obtained typically by applying artificial intelligence, e. g. using machine learning algorithms.

That is, according to the method of the present invention, it is possible to predict the survival rate or, in other words, a future risk of death based on the information on the peptidome/proteome of the individual, whereby the information, in a preferred embodiment, a scoring of a specific predetermined multidimensional classifier, is compared with information of the same peptidome/proteome present in a database. Based on the comparison of the scoring of the classifier, a survival rate of said individual for predetermined time periods or at the predetermined time end point can be predicted. This method for predicting the survival rate or a risk of death is particularly helpful in establishing preventive treatment, either by physical means or by pharmaceutical treatment.

The polypeptide markers or protein markers according to the present invention are proteins or peptides, including specific and defined (by their amino acid sequence) degradation products of proteins or peptides. These markers may be chemically modified, for example by posttranslational modifications, such as glycosylation, phosphorylation, hydroxylation, oxidation, alkylation, carbamylation, or disulphate bridges or by other reactions, for example, within the scope of the degradation. Apart from the parameters that determine the polypeptide markers, e. g. the molecular weight and migration time when applying a CE-MS method, it is possible to identify the sequence of the corresponding polypeptides by method known in the art, typically using tandem mass spectrometry.

As said, the method is based on the information obtained from the body fluid peptidome/proteome in the sample of the individual.

The present inventors recognized that applying the method of the present invention allows to determine a risk of death of the individual, although said individual may be apparently healthy or may suffer from a non-life threatening disease. The adverse event behind the risk of death may not yet be determined in said individual or may be connected with a pre-existing illness. On the other hand, the adverse event for a risk of death may be independently from a pre-existing illness. The predefined multidimensional classifier according to the present invention based on the body fluid, like the urinary peptidome/proteome allows to determine the survival rate or the risk of death of the individual when compared with data from a database. The data from the database may allow to classify individuals and into two individual groups of having a different risk of death.

In particular, the scoring of the classifier by comparing the information with information from a database may be by applying suitable algorithm, like an artificial intelligence routine. In this connection, the artificial intelligence routine may include a neural network (in general), like convolutional neural networks. For example, the neural network can be provided with trained weight-in factors or can be trained at use. For example, the multidimensional classifier is obtained with machine learning algorithms. That is, suitable methods and algorhitms include SVM, uMAP or decision trees. The peptide/protein marker of the peptidome/proteome present in the sample of the individual are combined into the classifier as described herein, whereby the peptides/proteins are associated with a future death. In an embodiment of the present invention, the specific predefined multidimensional classifier comprises at least three predetermined peptide/protein markers, for example, the classifier comprises at least five, at least ten, at least twenty, or at least thirty or more predetermined peptide/protein markers. As shown in the example below, the classifier contains more than seventy peptides, here seventy one peptides, allowing future prognosis of survival rate and death.

In an embodiment of the present invention, the steps of comparing the information and determining the survival rates according to step c) and d) described above may be conducted with artificial intelligence routine. As said above, the artificial intelligence routine may include a neural network. In an embodiment, the comparison of the information, in particular, of the classifier may be conducted by machine learning algorithm, in particular, by support vector machine.

The classifier according to the present invention is developed on the basis of data obtained from cohorts of subjects, including information on survival rate or risk of death based on the peptidome/proteome present in the sample.

In an embodiment, the sample is a urine sample and the peptidome/proteome is present in the urinary sample accordingly. For example, the urine sample is a midstream urine sample from the individual.

The classifier applied according to the present invention is developed typically from data of cohorts of subjects or individuals with known clinical history. The proteins/peptides included in the classifiers are significantly associated with future death or with a survival rate of the individual accordingly. As said, the multidimensional classifier may be developed using machine learning algorithms and may be further refined by optimising with a take one out procedure known to the skilled person as described in Wendt, R. et al., E Clinical Medicine 2021; 36:100883.

In an embodiment, this predefined classifier is applied onto the individual or a number of individuals for classifying these numbers of individuals into different groups or for predicting survival rate and risk of death of the individual and the cohort accordingly. Namely, a highly significant predictive power is demonstrated with the classifier enabling establishment of correlation between the risk of future death and the score obtained.

In this connection, the score is expressed as dimensionless numeric variable with known and defined association to the survival rate.

In an embodiment of the present invention, the method for the prediction of the survival rate of the individual includes the comparison by evaluating of a determined presence or absence or amplitude of the peptide/protein markers, for example, the classifier predefined composed of a multitude of predefined peptide/protein markers.

In an embodiment, the information of the peptidome/proteome, in particular, the predetermined group of peptide/protein markers include values for the molecular masses, migration time, in particular obtained by CE-MS, and/or the information includes an adjusted p-value for the association with future death or survival rate at predetermined time end points or within a predetermined time periods.

In an embodiment, the method according to the present invention comprises applying an artificial intelligence routine. Namely, the artificial intelligence routine is applied with information of the sequence of the peptidome/proteome, determined, in particular, of the predetermined peptide/protein markers as input data and information on the survival rate of said individual or the risk of future death as output information.

In an embodiment, the determination of the peptidome/proteome of the body fluid sample, in particular, the urinary sample is obtained by capillary electrophoresis, HPLC, gas-phase, ion spectrometry, in combination with mass spectrometry. The methods are well known to the skilled person accordingly.

In an embodiment of the present invention, the peptide/protein markers determined in the body fluid sample, are urinary peptide/protein markers including collagen-derived peptides/proteins, in particular, collagen α-1-derived peptides/proteins.

Further, in an embodiment of the present invention the method for prediction of the survival rate of an individual according to the present invention includes a prediction of an occurrence of possible diseases within the predetermined time period or at a predetermined time end point. That is, the occurrence of a disease may include an adverse health event. This adverse event may be an acute adverse event or may be a chronic adverse event. In addition, this adverse event may be a life threatening adverse event, in particular, may be an adverse event eventually resulting in death of the individual. The occurrence of the possible adverse event may be due to a pre-existing illness of the individual or may be due to a not yet determined illness of the individual or may be due to a new adverse event.

For example, the method according to the present invention may be applied for individuals under particular situations. For example, it may be of interest to receive information on the survival rate on persons or individuals within intensive care units (ICU) or with familiar predisposition of a specific disease or with genetic predisposition.

In an embodiment, the method according to the present invention for the prediction of the survival rate of an individual is a method wherein the individual may be a healthy individual or may be an individual afflicted with a known disease and the survival rate is reduced due to a different, not yet diagnosed disease.

In the method according to the present invention, the determination of the peptidome/proteome including a characterisation of the peptide/protein markers may be determined by means of capillary electrophoresis-mass spectrometry, a method which has been described in detail previously, see e. g. E. Mavrogeorgis et al., Molecules. 2021;26(23):7260.

Moreover, the database used according to the present invention contains data of a multitude of peptidome/proteome information of individuals either in form of the raw data or processed data, e. g. identifying specific peptides/proteins in combination with further information. For example, this information includes information on association with known adverse events including death.

The comparison may include a scoring of predefined peptide/protein markers which are summarized as classifier herein. The scoring, which is in a numerical value, allows to associate the score with the survival rate of the chance of death within a predetermined time period. The method will be detailed further by way of examples below.

In addition, the present invention relates to a computer implemented method for the prediction of the survival rate of an individual. Said method comprises the steps of
a) obtaining information of the peptidome/proteome in a sample obtained from the individual to be analysed;
b) Computing the information of step a) by comparing the information of the peptidome/proteome obtained in step a) with information on the peptidome/proteome obtained from a database; and
c) Determining the survival rate of said individual within a predetermined time period or a predetermined time end point, respectively.

Optionally, in a further step, the data of prediction of survival rate or the risk of death of the individual is shown on an output unit.

The computing according to step b) may include the application of suitable algorithms, like artificial intelligence routine as described for the method according to the present invention above. For example, the computing includes the generation of a scoring based on a predefined multidimensional classifier and comparing the score of said classifier with scores obtained from cohorts of individuals analysed and classified before. Analysis and classification is conducted particularly with respect to adverse events including death. Thus, the score obtained allows to determine the survival rate or the risk of death within a predetermined time period.

The computing may include suitable algorithms, like SVM, uMAP, decision trees, but also neural network with training algorithms, if required.

In a further embodiment of the present invention, a computer readable medium or computer program product having computer executable instructions for performing the steps according to the method of the present invention is provided.

The present invention will be described further by way of examples without limiting the same thereto.

### Example 1: Survival analysis in ICU patients

A urine sample is prepared according to standard protocol (1,2) and its proteome/peptidome contents are analysed. This resulted in a list of 1926 peptides and proteins with defined abundance in the sample.

In previous studies in a cohort of over 1000 subjects, a classifier predicting the risk of death within the next 100 days was developed. In this previous study, proteins/peptides significantly associated with future death were identified. These were combined into a high-dimensional classifier, using machine learning algorithms. The classifier was further refined by optimizing with a take-one-out procedure to contain 71 peptides (termed CD71):
Applying this classifier onto a cohort of 921 subjects admitted to the ICU, a highly significant predictive power could be demonstrated and enabled establishing a correlation between the risk of death within 100 days and the classification score.

To assess the risk of death of the subject giving the sample, the abundance information on the 71 predefined peptides is investigated:

| Protein/peptide ID / SEQ ID | Amplitude |
|---|---|
| 1. 99900220 | 0 |
| 2. 99900467 | 0 |
| 3. 99901399 | 0 |
| 4. 99902592 | 0 |
| 5. 99903025 | 0 |
| 6. 99904405 | 5645,55 |
| 7. 99904666 | 0 |
| 8. 99904862 | 3952,48 |
| 9. 99905044 | 659,44 |
| 10.99905259 | 1469,42 |
| 11.99905274 | 243,79 |
| 12.99905379 | 0 |
| 13.99905423 | 0 |
| 14.99905509 | 2160,81 |
| 15.99905551 | 0 |
| 16.99906176 | 0 |
| 17.99907275 | 1812,81 |
| 18.99907807 | 1693,2 |
| 19.99908755 | 1757,7 |
| 20.99908795 | 0 |
| 21.99908916 | 0 |
| 22.99909059 | 0 |
| 23.99910002 | 0 |
| 24.99910032 | 0 |
| 25.99910407 | 0 |
| 26.99910483 | 733,85 |
| 27.99910554 | 2236,03 |
| 28.99910883 | 0 |
| 29.99911002 | 0 |
| 30.99911193 | 997,78 |
| 31.99911415 | 0 |
| 32.99911559 | 0 |
| 33.99911777 | 0 |
| 34.99912101 | 379,81 |
| 35.99912211 | 357,38 |
| 36.99912366 | 0 |
| 37.99912411 | 0 |
| 38.99912661 | 0 |
| 39.99913200 | 0 |
| 40.99913322 | 0 |
| 41.99913825 | 0 |
| 42.99914211 | 0 |
| 43.99914445 | 0 |
| 44.99914876 | 0 |
| 45.99914887 | 387 |
| 46.99914893 | 0 |
| 47.99914935 | 0 |
| 48.99915015 | 2196 |
| 49.99915755 | 5339,07 |
| 50.99915778 | 0 |
| 51.99915883 | 0 |
| 52.99916170 | 585,65 |
| 53.99916177 | 0 |
| 54.99916611 | 0 |
| 55.99916615 | 0 |
| 56.99916966 | 0 |
| 57.99917047 | 613,55 |
| 58.99917221 | 0 |
| 59.99917280 | 0 |
| 60.99917288 | 0 |
| 61.99917419 | 0 |
| 62.99917505 | 0 |
| 63.99917596 | 0 |
| 64.99917626 | 0 |
| 65.99917690 | 0 |
| 66.99917825 | 0 |
| 67.99917890 | 0 |
| 68.99917947 | 0 |
| 69.99918831 | 0 |
| 70.99918906 | 0 |
| 71.99918909 | 0 |

Applying the CD71 classifier based on these 71 biomarkers results in a score of - 1.504, which reflects a chance of death within the next 100 days of 35.02%. This is a significantly increased risk, in comparison to all subjects in the validation study (average risk of death within 100 days was 23,13%). Based on this prediction, special attention should be given to this patient.

### Example 2: Survival analysis in a cohort of a multitude of subjects from various studies

A urine sample is prepared according to standard protocol and its proteome/peptidome contents are analysed as described, see below. This results in a list of 3920 peptides and proteins with defined abundance in the sample.

In previous studies in cohorts of over 1000 subjects, a classifier predicting the risk of death within the next 5 years was developed. In this previous study, proteins/peptides significantly associated with future death were identified. These were combined into a high-dimensional classifier, using machine learning algorithms. The classifier was further refined by optimizing with a take-one-out procedure to contain 71 peptides (termed CD71):
Applying this classifier onto a cohort of 8271 subjects demonstrated a highly significant predictive power and enabled establishing a correlation between the risk of future death and the classification score.

To assess the risk of death of the subject giving the sample, the abundance information on the 71 predefined peptides is investigated:

| Peptide/protein_ID | Amplitude |
|---|---|
| 1. 99900220 | 376,93 |
| 2. 99900467 | 459,22 |
| 3. 99901399 | 1375,51 |
| 4. 99902592 | 795,29 |
| 5. 99903025 | 5900,22 |
| 6. 99904405 | 0 |
| 7. 99904666 | 0 |
| 8. 99904862 | 440,81 |
| 9. 99905044 | 192,86 |
| 10.99905259 | 2467,51 |
| 11.99905274 | 2023,58 |
| 12.99905379 | 936,39 |
| 13.99905423 | 1284,7 |
| 14.99905509 | 0 |
| 15.99905551 | 5424,19 |
| 16.99906176 | 338,51 |
| 17.99907275 | 813,21 |
| 18.99907807 | 1255,48 |
| 19.99908755 | 1289,66 |
| 20.99908795 | 0 |
| 21.99908916 | 1078,22 |
| 22.99909059 | 601,7 |
| 23.99910002 | 0 |
| 24.99910032 | 2530,19 |
| 25.99910407 | 0 |
| 26.99910483 | 1897,03 |
| 27.99910554 | 8095,41 |
| 28.99910883 | 231,38 |
| 29.99911002 | 1913,47 |
| 30.99911193 | 1263,31 |
| 31.99911415 | 316,09 |
| 32.99911559 | 1267,45 |
| 33.99911777 | 146,72 |
| 34.99912101 | 656,51 |
| 35.99912211 | 588,78 |
| 36.99912366 | 454,24 |
| 37.99912411 | 343,73 |
| 38.99912661 | 4149,61 |
| 39.99913200 | 375,62 |
| 40.99913322 | 0 |
| 41.99913825 | 1135,47 |
| 42.99914211 | 635,96 |
| 43.99914445 | 163,97 |
| 44.99914876 | 641,34 |
| 45.99914887 | 0 |
| 46.99914893 | 0 |
| 47.99914935 | 58,85 |
| 48.99915015 | 0 |
| 49.99915755 | 0 |
| 50.99915778 | 105,04 |
| 51.99915883 | 147,94 |
| 52.99916170 | 2954,99 |
| 53.99916177 | 10106,78 |
| 54.99916611 | 0 |
| 55.99916615 | 0 |
| 56.99916966 | 1033,15 |
| 57.99917047 | 6484,27 |
| 58.99917221 | 49,54 |
| 59.99917280 | 943,09 |
| 60.99917288 | 1468,46 |
| 61.99917419 | 416,27 |
| 62.99917505 | 261,69 |
| 63.99917596 | 560,74 |
| 64.99917626 | 656,75 |
| 65.99917690 | 2383,49 |
| 66.99917825 | 257,55 |
| 67.99917890 | 825,93 |
| 68.99917947 | 567,78 |
| 69.99918831 | 201,86 |
| 70.99918906 | 295,19 |
| 71.99918909 | 127,29 |

Applying the CD71 classifier based on these 71 biomarkers results in a score of 1.601, which reflects a chance of death within the next 5 years of 3.44%. This is a significantly increased risk, in comparison to the general population at the same age (male, 51 years old). Adding additional relevant variables, age, blood pressure eGFR, LDL, HDL, blood glucose and number of comorbidities results in an adjusted risk of 3.41 %, still higher than the expected average.

The methods applied are based on standard protocols as identified, which are described for example in
1. Mavrogeorgis, E., Mischak, H., Latosinska, A., Siwy, J., Jankowski, V., and Jankowski, J. (2021) Molecules. 26***,***
2. Mischak, H., Vlahou, A., and loannidis, J. P. (2013) Clin. Biochem. 46, 432-443

## Claims

1. A method for the prediction of the survival rate of an individual within a predetermined time period or at a predetermined time end point, comprising the steps of
a) Providing a body fluid sample from the individual;
b) Determining the body fluid peptidome/proteome in the sample;
c) Comparing the information on the body fluid peptidome/proteome obtained in step b) with information on the body fluid peptidome/proteome present in a database;
d) Determining the survival rate of said individual within the predetermined time period or at the predetermined end point based on the comparison in step c).

2. The method for the prediction of the survival rate of an individual according to claim 1, wherein between step a) and step b) a reprocessing of the sample is conducted.

3. The method for the prediction of the survival rate of an individual according to any one of the preceding claims, wherein the step of comparison according to step c) includes the application of a specific predetermined multidimensional classifier, comprising at least 3, like at least 5, 10, 20 or 30 of predetermined peptide/protein markers.

4. The method for the prediction of the survival rate of an individual according to any one of the preceding claims, wherein at least one of steps c) and d) according to claim 1 are conducted with an artificial intelligence routine, including neural network.

5. The method for the prediction of the survival rate of an individual according to claim 4, wherein step c) is conducted by machine learning algorithm, in particular, support vector machine.

6. The method for the prediction of the survival rate of an individual according to any one of the preceding claims, wherein the comparison comprises an evaluation of a determined presence or absence or amplitude of peptide/protein markers, like of classifier based on a multitude of predetermined peptide/protein markers.

7. The method for the prediction of the survival rate of an individual according to any one of the preceding claims, wherein the information of the peptidome/proteome, in particular, the predetermined group of peptides/protein markers include values for the molecular masses, migration time, in particular, obtained by CE-MS and/or the information includes an adjusted p-value for the association with future survival rate at predetermined time points.

8. The method for the prediction of the survival rate of an individual according to any one of the preceding claims, wherein an artificial intelligence routine is applied with information of the sequence of the peptidome/proteome determined, in particular, of the predetermined peptide/protein markers as input data and information on the survival rate of said individual as output information.

9. The method for the prediction of the survival rate of an individual according to any one of the preceding claims, wherein said sample of the individual is a urine sample, like a midstream urine sample.

10. The method for the prediction of the survival rate of an individual according to any one of the preceding claims, wherein capillary electrophoresis, HPLC, gas-phase ion spectrometry in combination with mass spectrometry is used for determining the urinary peptidome/proteome, in particular, the peptide/protein markers.

11. The method for the prediction of the survival rate of an individual according to any one of the preceding claims, wherein the peptide/protein determined in the urinary peptidome/proteome includes collagen derived peptide/proteins, in particular, collagen α-1 derived-peptide/proteins.

12. The method for the prediction of the survival rate of an individual according to any one of the preceding claims, wherein the individual may be a healthy individual or may be an individual afflicted with a known disease and the survival rate may be reduced due to a different, not yet diagnosed disease.

13. A computer implemented method for the prediction of the survival rate of an individual, comprising the steps of
a) Obtaining information of the peptidome/proteome in a sample obtained from said individual;
b) Computing the information of step a) by comparing the information of the peptidome/proteome obtained in step a) with information on the peptidome/proteome obtained from a database; and
c) Determining the survival rate of said individual within a predetermined time period or a predetermined time end point, respectively;
d) Optionally, further comprising the step of showing the data of prediction of the survival rate of an individual on an output unit.

14. A computer readable medium or computer program product having computer executable instructions for performing the steps according to any one of claims 1 to 12.
